(19)

Europäisches
Patentamt
European
Patent Office
Office européen
des brevets

(11) **EP 3 342 545 A1**

(12) **EUROPEAN PATENT APPLICATION**
published in accordance with Art. 153(4) EPC

(43) Date of publication:
04.07.2018 Bulletin 2018/27

(21) Application number: 16838732.2

(22) Date of filing: 24.06.2016

(51) Int Cl.:
*B25J 3/00* (2006.01)     *B25J 13/00* (2006.01)
*B25J 19/06* (2006.01)

(86) International application number:
**PCT/JP2016/003067**

(87) International publication number:
**WO 2017/033380 (02.03.2017 Gazette 2017/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.08.2015 JP 2015165479**

(71) Applicant: **Kawasaki Jukogyo Kabushiki Kaisha
Kobe-shi, Hyogo 650-8670 (JP)**

(72) Inventors:
• **HASHIMOTO, Yasuhiko
Akashi-shi, Hyogo 673-8666 (JP)**
• **KAMON, Masayuki
Akashi-shi, Hyogo 673-8666 (JP)**

(74) Representative: **Müller-Boré & Partner
Patentanwälte PartG mbB
Friedenheimer Brücke 21
80639 München (DE)**

(54) **ROBOT SYSTEM**

(57)     A robot system (100) includes a robot (1) including a tactile sensor (5) and a hand (1c) having the tactile sensor, a robot controller (6) configured to control operation of the hand of the robot according to robot manipulating information, a manipulator (2), a tactile information processor (7) configured to generate tactile information defined by a pressure distribution based on pressures detected by at least the plurality of pressure sensors and spatial positions of the plurality of pressure sensors, convert the tactile information into sensible tactile information that is sensible by the operator, and output the sensible tactile information, a sensible tactile information presenting part (10) configured to present to the operator the sensible tactile information outputted from the tactile information processor.

FIG. 1

EP 3 342 545 A1

**Description**

TECHNICAL FIELD

The present disclosure relates to a robot system.

BACKGROUND ART

Conventionally, robot control systems utilizing a tactile sense are known. For example, a manipulating device which manipulates a mobile robot, while obtaining a force feedback between a joystick and the mobile robot, is known (e.g., see Patent Document 1). Moreover, a robot hand provided with a tactile sensor system is known (e.g., see Patent Document 2). Moreover, a pressure-distribution information detecting device which is provided with tactile sensors at a robot hand thereof, and controls the robot hand based on a pressure distribution detected by the tactile sensors, is known (e.g., see Patent Document 3).

[Reference Documents of Conventional Art]

[Patent Documents]

[Patent Document 1] JP2009-282720A
[Patent Document 2] JP2014-145717A
[Patent Document 3] JP2014-145717A

DESCRIPTION OF THE DISCLOSURE

[Problems to be Solved by the Disclosure]

However, these conventional technologies still have room for an improvement in terms of practicality.
One purpose of the present disclosure is to improve practicality of a robot system in which the robot is remotely controlled based on tactile information defined by a pressure distribution.

[Summary of the Disclosure]

The present inventors have diligently examined the problems described above in order to solve them. The present inventors have focused on a work which is performed by a human utilizing not a "force sensation" that is simply a force or pressure, but an essential "tactile sense," i.e., senses, such as a degree of smoothness (uneven or irregular feel), a degree of friction (rough feel or smooth feel), etc. Such a work may be, for example, a work in which, when repairing a damaged member by smoothening the damaged surface, the damaged surface is finished while checking the degree of smoothness of the damaged surface with the tactile sense of a hand, a work in which the degree of smoothness of the surface of a member is inspected with the tactile sense of the hand, etc. The degree of smoothness (uneven feel) and the degree of friction (rough feel or smooth feel) are tactile senses defined by a pressure distribution which the human senses.
On the other hand, the manipulating device disclosed in Patent Document 1 controls using the "force sensation," but does not use the essential "tactile sense." The robot hand disclosed in Patent Document 2 uses the "tactile sense" defined by the pressure distribution, but it is difficult to control the operation of the robot hand exactly like the human performing the work because an arithmetic processor controls the operation of the robot hand based on detection outputs of the tactile sensors. The pressure-distribution information detecting device disclosed in Patent Document 3 is also difficult to control the operation of the robot hand exactly like the human performing the work because a behavior control device controls the operation of the robot hand based on the pressure distribution detected the tactile sensors similar to the robot hand disclosed in Patent Document 2.
Therefore, the inventors have arrived at that a human judges operation to be performed by a robot hand based on detection outputs of a tactile sensors provided to a robot hand, and instructs exact operation to the robot hand. This enables the operation of the robot hand to be controlled exactly like the human performing the work.
Therefore, a robot system according to one aspect of the present disclosure includes a robot including a tactile sensor having a plurality of pressure sensors arranged at mutually different spatial positions, and a hand having the tactile sensor, a robot controller configured to control operation of the hand of the robot according to robot manipulating information, a manipulator configured, when an operator operates the manipulator to manipulate the hand of the robot, to transmit to the robot controller the robot manipulating information according to the operation, a tactile information processor configured to generate tactile information defined by a pressure distribution based on pressures detected at least the plurality of pressure sensors and spatial positions of the plurality of pressure sensors, convert the tactile information into sensible tactile information that is sensible by the operator, and output the sensible tactile information, and a sensible tactile information presenting part configured to present to the operator the sensible tactile information outputted from the tactile information processor.

With this configuration, when the tactile sensor having the plurality of pressure sensors arranged at mutually different spatial positions detects an object at the hand of the robot, the tactile information processor generates the tactile information defined by the pressure distribution based on the pressures detected by at least the plurality of pressure sensors and the spatial positions of the plurality of pressure sensors, converts the tactile information into the sensible tactile information that is sensible by the operator, and outputs the sensible tactile information. Then, the sensible tactile information presenting part presents to the operator the sensible tactile information outputted from the tactile information processor. The operator grasps the tactile sense detected by the tactile sensor from the sensible tactile information, and operates the manipulator to manipulate the robot so that the robot performs a suitable work. Then, the manipulator transmits the robot manipulating information according to the manipulation to the robot controller, and the robot controller controls the operation of the hand of the robot according to the robot manipulating information. Thus, the operator judges the operation to be performed by the hand of the robot based on the detection output of the tactile sensor which the hand of the robot has. Since exact operation is able to be instructed to the hand of the robot, the operation of the hand of the robot is controllable exactly like the human performing the work. As a result, a practicality of the robot system in which the robot is remotely controlled based on the tactile information defined by the pressure distribution is improved.

The tactile sensor may include a base body and a plurality of pressure sensors fixed to the surface of the base body at given positions. The tactile information processor may generate the tactile information defined by the pressure distribution based on the pressures detected by the plurality of pressure sensors, and the given positions of the plurality of pressure sensors in the base body.

With this configuration, the degree of smoothness is detectable as the tactile sense.

The tactile sensor may include a base body, a plurality of pressure sensors fixed to the surface of the base body at given positions, and one or more position sensors fixed to the base body corresponding to the plurality of pressure sensors. The tactile information processor may generate the tactile information defined by a time change of the pressure distribution based on pressures detected and by the plurality of pressure sensors of, and spatial positions detected by the one or more position sensors.

With this configuration, the degree of smoothness or the degree of friction is detectable as the tactile sense.

The tactile information may be one of a degree of smoothness and a degree of friction.

The sensible tactile information may be sensed information defined so that a degree of sense by the operator changes corresponding to a degree of tactile in the tactile information.

With this configuration, the operator is able to grasp with the simple configuration the degree of tactile detected by the tactile sensor based on the degree of sense of the sensed information.

The sensed information may be one of visual information, hearing information, and vibration, and the sensible tactile information presenting part may be one of a display, a speaker, and an actuator.

With this configuration, the operator is able to suitably grasp the degree of tactile detected by the tactile sensor.

The manipulator may be a master arm, and an arm including the hand of the robot is a slave arm. The slave arm may be manipulated by the master arm.

With this configuration, the operator is able to suitably control the operation of the hand of the robot. Further, the slave arm is configured to be switchable among an automatic mode, a hybrid mode, and a manual mode so that a non-stopping robot is implemented by switching the mode to the hybrid mode or the manual mode when a trouble occurs.

[Effect of the Disclosure]

The present disclosure is configured as described above, and is capable of improving the practicality of the robot system which remotely controls the robot based on the tactile information defined by the pressure distribution.

BRIEF DESCRIPTION OF DRAWINGS

Fig. 1 is a schematic diagram illustrating one example of a configuration of a robot system according to Embodiment 1 of the present disclosure.
Figs. 2(a) and (b) are views illustrating one example of a configuration of a tactile sensor used for the robot system of Fig. 1, where (a) is a plan view and (b) is a side view.

Figs. 3(a) to (c) are views illustrating repairing states of a damaged member, and where (a) is a view illustrating a damaged state, (b) is a view illustrating an under-repair state, and (c) is a view illustrating repair-completed state.

Figs. 4(a) to (c) are views schematically illustrating sensible tactile information based on a detection output of the tactile sensor of a damaged surface of the damaged member of Fig. 3, where (a) is a view corresponding to the state of Fig. 3(a), (b) is a view corresponding to the state of Fig. 3(b), and (c) is a view corresponding to the state of Fig. 3(c).

Figs. 5(a) to (c) are views illustrating a generation principle of the sensible tactile information in a robot system according to Embodiment 2 of the present disclosure, where (a) is a view illustrating a histogram of the pressure level distribution of Fig. 4(a), (b) is a view illustrating a histogram of the pressure level distribution of Fig. 4(b), and (c) is a view illustrating a histogram of the pressure level distribution of Fig. 4(c).

Fig. 6 is a view illustrating a relation between a standard deviation of the pressure level distribution and a sensing level, in the sensible tactile information in the robot system according to Embodiment 2 of the present disclosure.

Figs. 7(a) and (b) are views illustrating one example of a configuration of a tactile sensor used for a robot system according to Embodiment 3 of the present disclosure, where (a) is a plan view and (b) is a side view.

Fig. 8 is a schematic diagram illustrating one example of a configuration of a robot system according to Embodiment 4 of the present disclosure.

MODES FOR CARRYING OUT THE DISCLOSURE

Hereinafter, embodiments of the present disclosure will be described with reference to the accompanying drawings. Note that, below, the same reference characters are given to the same or corresponding elements throughout the figures to omit redundant description.

(Embodiment 1)

[Configuration]

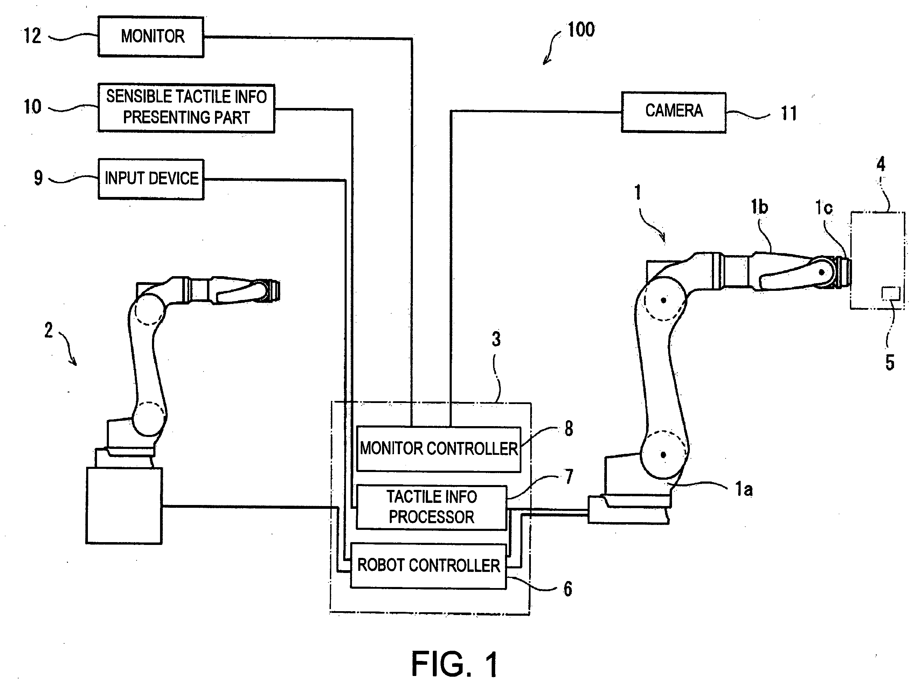

Fig. 1 is a schematic diagram illustrating one example of a configuration of a robot system according to Embodiment 1 of the present disclosure.

Referring to Fig. 1, a robot system 100 of Embodiment 1 is comprised of, for example, a master-slave type robot system.

The robot system 100 includes a slave arm 1 comprised of a first robot, a master arm 2 comprised of a second robot, a control device 3, a tactile sensor 5, an input device 9, a camera 11, and a monitor 12. The slave arm 1 may be comprised of a robot of any type. In Embodiment 1, the slave arm 1 is comprised of, for example, a well-known articulated robot, which includes a pedestal 1a, an articulated arm 1b provided to the pedestal 1a, and a wrist 1c provided to a tip end of the arm 1b. An end effector 4 is attached to the wrist 1c. The wrist 1c and the end effector 4 constitute a hand of the slave arm 1. The tactile sensor 5 is attached to the end effector 4.

The master arm 2 may be comprised of a robot of any type. This robot may be, for example, a simple device like a control lever, as long as it is capable of operating the slave arm 1 by the operator manipulating the robot. When the operator manipulates the hand of the slave arm 1, the master arm 2 transmits manipulating information (robot manipulating information) according to the manipulation to a robot controller 6 described later.

The control device 3 includes the robot controller 6, a tactile information processor 7, and a monitor controller 8. The control device 3 is comprised of a device having arithmetic processing capabilities, such as a computer, a microcontroller, and a microprocessor. The robot controller 6, the tactile information processor 7, and the monitor controller 8 are implemented by an arithmetic processor (not illustrated) of the control device 3 executing a given program stored in a memory part (not illustrated) of the control device 3. The hardware configuration of the control device 3 is arbitrary, and the control device 3 may be provided independently from other devices of the slave arm 1 etc., or may be provided integrally with other devices.

The input device 9 is comprised of man-machine interface(s), such as a touch panel and/or a keyboard. The input device 9 is mainly used in order to switch three modes of an automatic mode, a hybrid mode, and a manual mode of the slave arm 1, and input various data, etc. The information inputted into the input device 9 is transmitted to the robot controller 6.

The robot controller 6 controls operation of the slave arm 1 based on the information inputted into the input device 9 and the manipulating information transmitted from the master arm 2. Here, those information are suitably stored in the memory part of the control device 3.

The camera 11 is provided to be capable of imaging the operation of the slave arm 1 within all or part of a movable range of the slave arm 1. The image information imaged by the camera 11 is transmitted to the monitor controller 8. The monitor controller 8 controls the monitor 12 to display an image corresponding to the image information.

By the above configuration, the operator is able to operate the master arm 2 while looking at the monitor 12 to manipulate the slave arm 1 as he/she intended.

The tactile sensor 5 has a plurality of pressure sensors arranged at mutually different spatial positions, and pressures detected by the plurality of pressure sensors are transmitted to the tactile information processor 7, respectively. The tactile information processor 7 generates tactile information defined by a pressure distribution based on pressures detected by the plurality of these pressure sensors and the spatial positions of the plurality of pressure sensors, and converts the tactile information into sensible tactile information which is sensible by the operator. Then, the sensible tactile information is transmitted (outputted) to a sensible tactile information presenting part 10. The sensible tactile information presenting part 10 presents the operator the sensible tactile information transmitted from the tactile information processor 7. Specifically, the sensible tactile information is sensed information defined so that a degree of sense by the operator changes corresponding to a degree of tactile in the tactile information. Concrete examples of this will be described later. Thus, the operator is capable of grasping with the simple configuration the degree of tactile detected by the tactile sensor 5 of the slave arm 1 based on the degree of sense of the sensed information. The sensed information is visual information, hearing information, or tactile information. The sensible tactile information presenting part 10 is, for example, a display, a speaker, or an actuator. According to this configuration, the operator is capable of suitably grasping the degree of tactile detected by the tactile sensor 5.

Note that the pressures detected by the plurality of pressure sensors of the tactile sensor 5 are also transmitted to the robot controller 6. When the tactile sensor 5 is manipulated by the master arm 2 so as to be lowered, the robot controller 6 stops lowering of the tactile sensor 5 in response to a detection of a given maximum pressure Pmax by any of the plurality of pressure sensors of the tactile sensor 5.

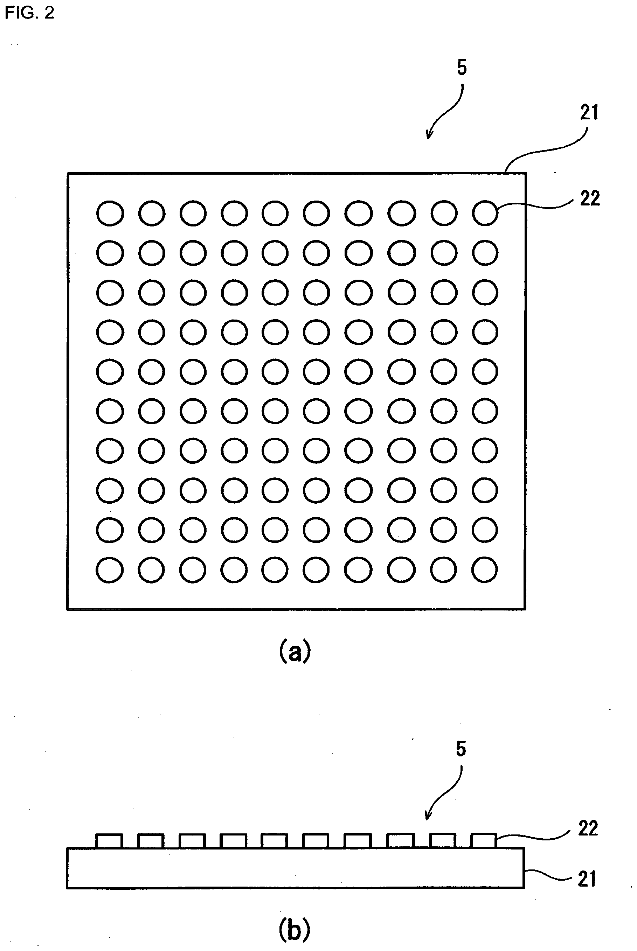

Figs. 2(a) and (b) are views illustrating one example of a configuration of the tactile sensor 5, where (a) is a plan view and (b) is a side view.

Referring to Fig. 2, the tactile sensor 5 includes a base body 21 and a plurality of pressure sensors 22. Although the number of pressure sensors 22 is, for example, 100, but may be any number as long as it is two or more. Although the shape of the base body 21 is arbitrary, the base body 21 is comprised of, for example, a tetragon plate-like member. The material of the base body 21 may be plastic, metal, or rubber. The plurality of pressure sensors 22 are arranged, for example, on one of the principal surfaces of the base body 21 in a matrix. Note that the arrangement of the plurality of pressure sensors 22 is arbitrary. The positions of the plurality of pressure sensors on the principal surface of the base body 21 are stored in the memory part (not illustrated) of the control device 3. The tactile information processor 7 described above generates the tactile information defined by the pressure distribution based on the pressures detected by the plurality of pressure sensors 22 arranged in the matrix, and the positions of the plurality of pressure sensors on the principal surface of the base body 21 stored in the memory part of the control device 3.

[Operation]

Next, operation of the robot system configured as described above is described using Figs. 1 to 4. Here, a work in which, when repairing a member which is damaged (hereinafter, referred to as the "damaged member") by smoothening the damaged surface, the damaged surface is finished while checking a degree of smoothness of the damaged surface by the tactile sensor 5 of the hand of the slave arm 1, is described as an example. Such a damaged member may be a component of a workpiece damaged during being processed, or a damaged member other than the workpiece, which requires a repair. Moreover, it is assumed that a degree of irregularity of the damaged surface of the damaged member is difficult for the operator to judge through the monitor 12 due to the resolution and/or the camera angles of the camera 11. Moreover, it is assumed that the operating mode of the slave arm 1 is the automatic mode, a trouble occurred during the automatic mode and the damaged member is generated, and the operating mode can be resumed to the automatic mode by repairing the damaged member.

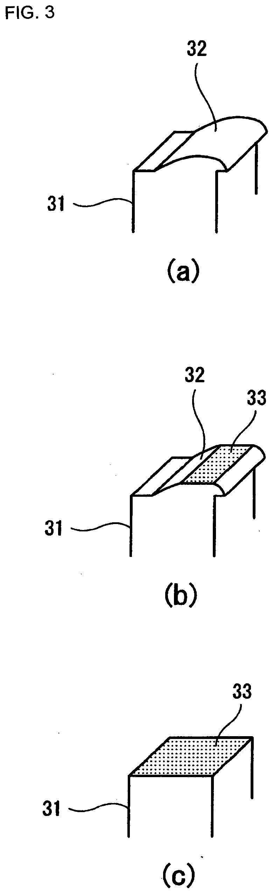

Figs. 3(a) to (c) are views illustrating repairing states of a damaged member 31, where (a) is a view illustrating a damaged state, (b) is a view illustrating an under-repair state, and (c) is a view illustrating a repair-completed state.

Referring to Figs. 1 to 4, the end effector 4 to which a surface grinder (not illustrated) and the tactile sensor 5 are fixed is attached to the wrist 1c of the slave arm 1. The surface grinder is fixed so that its axis of rotation is oriented vertically. The tactile sensor 5 is fixed so that the principal surface of the base body 21 where the pressure sensors 22 are provided is oriented downwardly.

As illustrated in Figs. 3(a) to (c), the damaged member 31 is fixed by an appropriate jig so that the damaged surface 32 is located at an upper end and the damaged member 31 extends vertically.

The operator inputs by the input device 9 an instruction for switching the operating mode of the slave arm 1 to the manual mode. Then, the robot controller 6 switches the operating mode of the slave arm 1 to the manual mode.

Next, while the operator is looking at the monitor 12, he/she manipulates the master arm 2 so that the surface grinder and the tactile sensor 5 which are fixed to the end effector 4 of the slave arm 1 are alternately pressed against the damaged surface 32 of the damaged member 31 from above. Specifically, the operator first presses the tactile sensor 5 against the damaged surface 32 of the damaged member 31 from above. Then, the tactile sensor 5 transmits to the tactile information processor 7 the pressures which are respectively detected by the plurality of (here, 100) pressure sensors 22. The tactile information processor 7 generates the tactile information defined by the pressure distribution based on the pressures which are respectively detected by the plurality of pressure sensors 22 of the tactile sensor 5, and the positions of the plurality of pressure sensors 22 on the principal surface of the base body 21 stored in the memory part of the control device 3. Here, "generating the tactile information defined by the pressure distribution" means that, specifically, associating (corresponding) the pressures which are respectively detected by the plurality of pressure sensors 22 of the tactile sensor 5 with (to) the positions of the plurality of pressure sensors 22 on the principal surface of the base body 21 stored in the memory part of the control device 3. When such an association (correspondence) is performed, "the tactile information defined by the pressure distribution" is generated. Then, the tactile information processor 7 converts the "tactile information defined by the pressure distribution" into the sensible tactile information which is sensible by the operator.

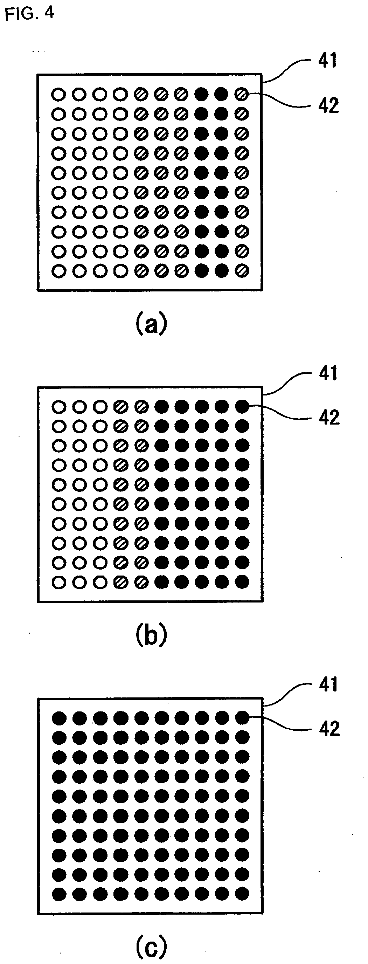

Figs. 4(a) to (c) are conceptual diagrams schematically illustrating the sensible tactile information based on the detection output of the tactile sensor 5 of the damaged surface 32 of the damaged member 31 of Fig. 3, where (a) is a view corresponding to the state of Fig. 3(a), (b) is a view corresponding to the state of Fig. 3(b), and (c) is a view corresponding to the state of Fig. 3(c).

In the conceptual diagrams of Figs. 4(a) to (c), a tetragon 41 represents the base body 21 of the tactile sensor 5, and circles 42 represents the pressure sensors 22 of the tactile sensor 5. Gradation of the circles 42 represents a pressure level. As described above, when any of the plurality of pressure sensors 22 of the tactile sensor 5 detects the given maximum pressure Pmax, the robot controller 6 stops the lowering of the tactile sensor 5. The tactile information processor 7 ranks the pressures P detected by the plurality of pressure sensors 22 of the tactile sensor 5, for example, into three pressure levels PL1-PL3. Note that the number of pressure levels is not limited to 3, but is arbitrary. Specifically, for example, the pressure P is ranked as follows:

$$0 \leq PL1 < Pmax / 3$$

$$Pmax / 3 \leq PL2 < 2Pmax / 3$$

$$2Pmax / 3 \leq PL3 < Pmax$$

In Figs. 4(a) to (c), "white (blank)," "gray (hatched)," and "black (blotted out)" of the circles 42 represent the "pressure level PL1," "pressure level PL2," and "pressure level PL3," respectively.

The tactile information processor 7 generates the image (sensed information) like the conceptual diagrams illustrated in Figs. 4(a) to (c) based on such sensible tactile information, and causes the sensible tactile information presenting part 10 to present the image. Specifically, the sensible tactile information presenting part 10 is, for example, a display, and an image like the conceptual diagrams illustrated in Figs. 4(a) to (c) is displayed on the display. The operator who saw the image is able to sense differently in the sensed degree according to the gradation of the circle 42 in the image, and sense the degree of tactile (pressure) detected by the tactile sensor 5. Specifically, in the image, the operator senses a larger degree of irregularity of the damaged surface 32 of the damaged member 31 as a variation of the circles 42 in the color becomes larger. Further, since the image contains the positional information on the pressure-sensitive points, the operator who saw the image is able to instinctively sense the pressure distribution in the tactile sensor 5.

Next, description returns to the repairing work of the damaged member 31. As described above, when the operator presses the tactile sensor 5 against the damaged surface 32 of the damaged member 31 from above, the image of the conceptual diagram illustrated in Fig. 4(a) is displayed on the sensible tactile information presenting part 10 which is the display. Since the variation in the color of the circles 42 is large in the image, the operator judges that the degree of irregularity of the damaged surface of the damaged member 31 is large. Then, the operator pushes the surface grinder against the damaged surface 32 of the damaged member 31 from above, and continues pressing for a given period of time, and after that, stops pressing of the surface grinder. Note that it may be set beforehand so that the robot controller 6 automatically controls the time of grinding by the surface grinder and a lowering distance. Fig. 3(b) illustrates a state of the damaged surface 32 of the damaged member 31 at this time. The reference character 33 indicates a ground surface which is ground by the surface grinder. Then, the operator presses the tactile sensor 5 against the damaged surface 32 of the damaged member 31 from above. Then, the image of the conceptual diagram illustrating in Fig. 4(b) is displayed on the sensible tactile information presenting part 10. Since the variation of the circles 42 in the color is still large in this image, the operator judges that the degree of irregularity of the damaged surface of the damaged member 31 is still large. Then, the operator presses the surface grinder against the damaged surface 32 of the damaged member 31 from above, and continues pressing for the given period of time, and after that, stops pressing of the surface grinder. Fig. 3(c) illustrates a state of the upper end face of the damaged member 31 at this time. All the damaged surfaces are removed by grinding from the upper end face of the damaged member 31 at this time, and become the ground surface 33. Then, the operator presses the tactile sensor 5 against the damaged surface (upper end face) of the damaged member 31 from above. Then, the image of the conceptual diagram illustrating in Fig. 4(c) is displayed on the sensible tactile information presenting part 10. Since all the colors of the circles 42 in this image are black, the operator judges that the irregularity of the damaged surface of the damaged member 31 is substantially eliminated, and ends the repairing.

Then, the operator inputs by the input device 9 an instruction for switching the operating mode of the slave arm 1 to the automatic mode. Then, the robot controller 6 switches the operating mode of the slave arm 1 to the automatic mode. Thus, even if a trouble occurs, stopping of the operation of the slave arm 1 is avoided.

As described above, according to Embodiment 1, the operator is able to judge the operation to be performed by the hand of the slave arm 1 based on the detection output of the tactile sensor 5 which the hand of the slave arm 1 has. Since exact operation is able to be instructed to the hand of the slave arm 1, the operation of the hand of the slave arm 1 is controllable exactly like the human performing the work. As a result, the practicality of the robot system in which the robot is remotely controlled based on the tactile information defined by the pressure distribution is improved. Moreover, since stopping of the operation of the slave arm 1 is avoided even if a trouble occurs, a non-stopping robot can be implemented.

(Embodiment 2)

Embodiment 2 of the present disclosure is different from Embodiment 1 in the sensible tactile information, and others are the same as Embodiment 1.

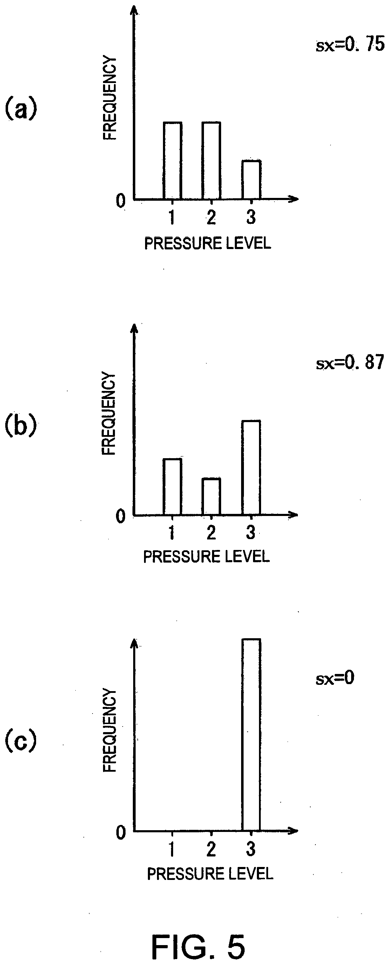

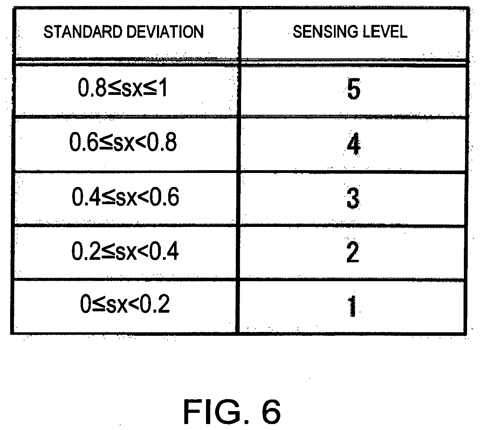

Figs. 5(a) to (c) are views illustrating a principle of generating sensible tactile information in a robot system according to Embodiment 2 of the present disclosure, where (a) is a view illustrating a histogram of the pressure level distribution of Fig. 4(a), (b) is a view illustrating a histogram of the pressure level distribution of Fig. 4(b), and (c) is a view illustrating a histogram of the pressure level distribution of Fig. 4(c). Fig. 6 is a view illustrating a relation between a standard deviation of the pressure level distribution and the sensing level in the sensible tactile information in the robot system according to Embodiment 2 of the present disclosure.

In Embodiment 2, the tactile information processor 7 generates simplified sensible tactile information without containing the positional information on the pressure-sensitive points based on the sensible tactile information in Embodiment 1 described above. This principle is described. Figs. 4(a) to (c) illustrate, precisely, distributions of the pressure level of the pressures P detected by the plurality of pressure sensors 22, respectively. The variation in the pressure level of the pressure P (e.g., a standard deviation) has a magnitude according to the degree (magnitude) of irregularity of the surface of the tactile sensor 5, which is to be detected. Thus, the tactile information processor 7 gives numerical values of "1," "2" and "3" to the pressure levels PL1, PL2 and PL3 in the sensible tactile information in Embodiment 1, respectively. Then, the histograms of the distribution of the pressure level of Figs. 4(a) to (c) become the histograms illustrated in Figs. 5(a) to (c), respectively. The standard deviation sx in the histograms of Figs. 5(a) to (c) is "0.75," "0.87" and "0," respectively. Note that, here, the standard deviation sx takes a value of $0 \leq sx \leq 1$. When comparing the histograms and the standard deviations sx illustrated in Figs. 5(a) to (c) with the degree of irregularity of the damaged surface 32 of the damaged member 31 illustrated in Figs. 3(a) to (c), it should be understood that one suitably corresponds to the other.

Then, the tactile information processor 7 corresponds the standard deviation sx to the operator's sensing level. Specifically, the tactile information processor 7 ranks the operator's sensing level into, for example, five levels of CL1-CL5 as illustrated in Fig. 6, and corresponds the standard deviation sx to the five levels. Note that the number of ranks of the sensing level is not limited to five, but is arbitrary. In fact, the tactile information processor 7 directly calculates the rank and the standard deviation sx of the pressure levels by using the pressures respectively detected by the plurality of pressure sensors 22 (it does not generate the histogram). Note that the ranking of the pressure level may be omitted, but the standard deviation sx may be directly calculated from the pressures respectively detected by the plurality of pressure sensors 22.

Then, the tactile information processor 7 transmits the sensed information signals of these five levels to the sensible tactile information presenting part 10. If the sensible tactile information presenting part 10 is the display, the speaker, or the actuator provided to a seat on which the operator sits, the sensible tactile information presenting part 10 presents (outputs) to the operator, for example, a screen of brightness according to the level of the sensed information signals, sound of volume according to the level of the sensed information signals, or vibration of amplitude according to the level of the sensed information signals, respectively.

According to such Embodiment 2, the operator is able to suitably grasp with the simple configuration the degree of tactile detected by the tactile sensor 5 of the hand of the slave arm 1 based on the degree of sense of the sensed information.

(Embodiment 3)

Embodiment 3 of the present disclosure is different in the tactile sensor 5 from the tactile sensor 5 of Embodiment 1, and others are the same as those of Embodiment 1.

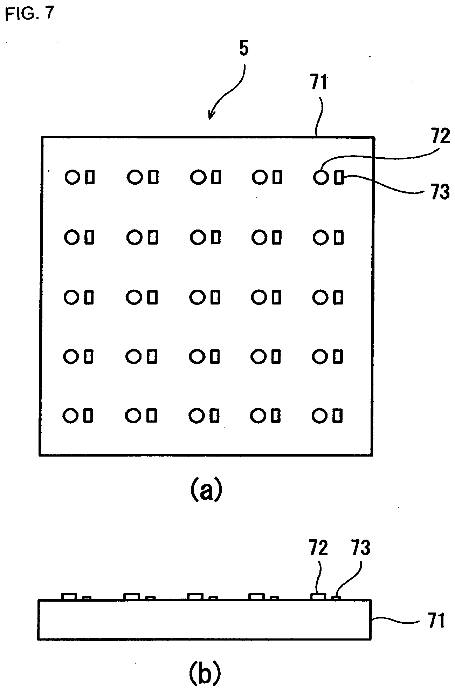

Figs. 7(a) and (b) are views illustrating one example of a configuration of the tactile sensor used for the robot system according to Embodiment 3 of the present disclosure, where (a) is a plan view and (b) is a side view.

Referring to Fig. 7, in Embodiment 3, the tactile sensor 5 includes a base body 71, a plurality of pressure sensors 72 fixed to the surface of the base body 71 at given positions, and one or more position sensors 73 fixed to the base body 71 corresponding to the plurality of pressure sensors 22. Specifically, the base body 71 is comprised of, for example, a tetragon plate-like member. Note that the shape of the base body is arbitrary. The material of the base body 71 may be plastic, metal, or rubber.

The pressure sensor 72 and the position sensor 73 are arranged so that, for example, pair of the pressure sensor 72 and the position sensor 73 are distributed in a matrix on one of the principal surfaces of the base body 71. Standard positions of the pressure sensors 72 and the position sensors 73 on the principal surface of each base body 71 are stored in the memory part of the control device 3. Here, the "standard position" means the position in a state where the base body 71 is not deformed. The position sensor may be, for example, a displacement sensor. The displacement sensor may be, for example, an acceleration sensor, a gyroscope sensor, etc.

The tactile sensor 5 configured in this way is deformed due to a frictional force with the surface to be detected when the tactile sensor 5 is moved in a direction parallel to the principal surface in a state where the tactile sensor 5 is pressed against the surface to be detected in the thickness direction at a given pressure. In this case, a degree of deformation is detected by the position sensor 73, and a pressure distribution in the tactile sensor 5 is also detected. The tactile information processor 7 generates tactile information which is a degree of friction by the degree of deformation and a time change of the pressure distribution. The relation between "the degree of deformation and the time change of the pressure distribution," and "the degree of friction" is determined beforehand according to experiments, simulations, etc.

Since subsequent processing of the tactile information is similar to those of Embodiments 1 and 2, description thereof is omitted. Note that, if the tactile sensor 5 is not moved in the direction parallel to the principal surface, the degree of irregularity of the surface to be detected is detectable similar to Embodiment 1.

According to such Embodiment 3, the degree of smoothness or the degree of friction is detectable as the tactile sense.

(Embodiment 4)

Embodiment 4 of the present disclosure is different from Embodiment 1 in that the robot system is provided with a normal robot and a manipulator which remotely controls the normal robot, and others are similar to those of Embodiment 1.

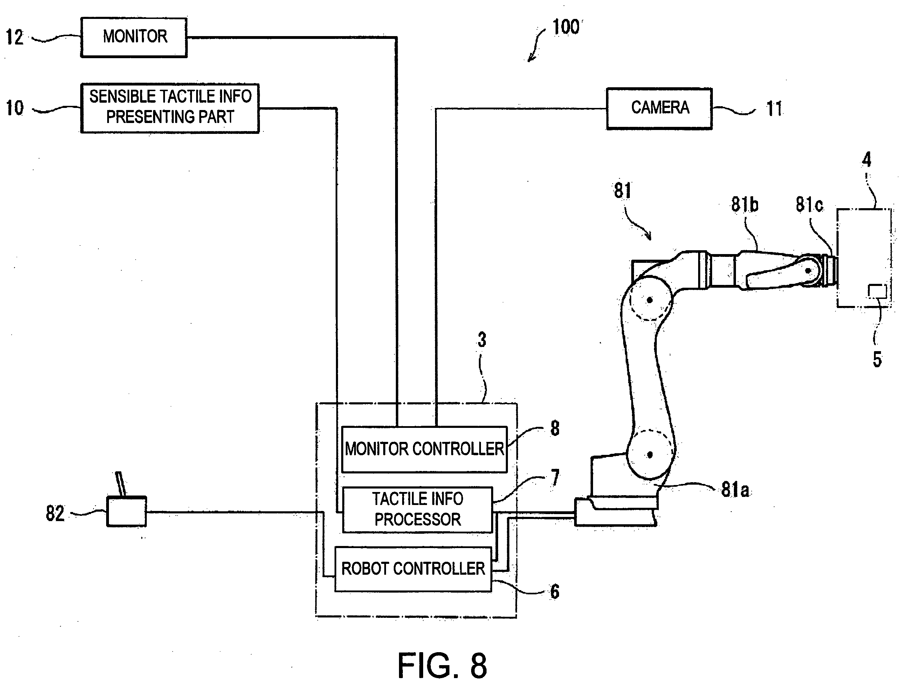

Fig. 8 is a schematic diagram illustrating one example of a configuration of the robot system according to Embodiment 4 of the present disclosure.

Referred to Fig. 8, in Embodiment 4, the robot system 100 of Embodiment 4 is provided with, for example, a common robot 81 and a manipulator 82 for manually operating the robot 81. The type of the robot 81 is not limited in particular. Here, the robot 81 is, for example, an articulated robot. Reference characters 81a, 81b and 81c illustrate a pedestal, an arm, and a wrist, respectively. The manipulator 82 is comprised of, for example, a well-known job stick. Note that the input device 9 of Embodiment 1 is omitted.

Since the configurations are the same as Embodiment 1 other than the configurations described above, descriptions thereof are omitted.

According to such Embodiment 4, the robot system of the present disclosure is applicable to a common robot system in which a robot is remotely controlled by manual operation.

(Other Embodiments)

In Embodiment 1, instead of repairing work of the damaged surface 32 of the damaged member 31, a work to inspect the degree of smoothness of the surface of the member by the tactile sensor 5 of the hand of the slave arm 1 may be performed.

In Embodiments 1 to 4, the operator may sense the member indirectly through the tactile sensor 5 of the hand of the robot 81 or the slave arm 1, and the operator may manipulate the robot 81 or the slave arm 1 based on the sense to perform the work utilizing the detection output of the tactile sensor 5.

It is apparent for a person skilled in the art that many improvements and other embodiments of the present disclosure are possible from the above description. Therefore, the above description is to be interpreted only as illustration, and it is provided in order to teach a person skilled in the art the best mode which implements the present disclosure. Details of the structures and/or the functions may be substantially changed without departing from the spirit of the present disclosure.

INDUSTRIAL APPLICABILITY

The robot system of the present disclosure is useful as the robot system with an improved practicality.

DESCRIPTION OF REFERENCE CHARACTERS

| 1 | Slave Arm |
| 2 | Master Arm |
| 3 | Control Device |
| 4 | End Effector |
| 5 | Tactile Sensor |
| 6 | Robot Controller |
| 7 | Tactile Information Processor |
| 8 | Monitor Controller |
| 9 | Input Device |
| 10 | Sensible Tactile Information Presenting Part |
| 11 | Camera |
| 12 | Monitor |
| 21 | Base Body |
| 22 | Pressure Sensor |
| 31 | Damaged Member |
| 32 | Damaged Surface |
| 33 | Ground Surface |
| 41 | Tetragon |
| 42 | Circle |
| 71 | Base Body |
| 72 | Pressure Sensor |
| 73 | Position Sensor |
| 81 | Robot |
| 82 | Manipulator |

**Claims**

1.  A robot system, comprising:

   a robot including a tactile sensor having a plurality of pressure sensors arranged at mutually different spatial positions, and a hand having the tactile sensor;
   a robot controller configured to control operation of the hand of the robot according to robot manipulating information;
   a manipulator configured, when an operator operates the manipulator to manipulate the hand of the robot, to transmit to the robot controller the robot manipulating information according to the operation;
   a tactile information processor configured to generate tactile information defined by a pressure distribution based on pressures detected by at least the plurality of pressure sensors and spatial positions of the plurality of pressure sensors, convert the tactile information into sensible tactile information that is sensible by the operator, and output the sensible tactile information; and
   a sensible tactile information presenting part configured to present to the operator the sensible tactile information outputted from the tactile information processor.

**2.** The robot system of claim 1, wherein,
the tactile sensor includes a base body and a plurality of pressure sensors fixed to the surface of the base body at given positions, and
the tactile information processor generates the tactile information defined by the pressure distribution based on the pressures detected by the plurality of pressure sensors, and the given positions of the plurality of pressure sensors in the base body.

**3.** The robot system of claim 1, wherein,
the tactile sensor includes a base body, a plurality of pressure sensors fixed to the surface of the base body at given positions, and one or more position sensors fixed to the base body corresponding to the plurality of pressure sensors, and
the tactile information processor generates the tactile information defined by a time change of the pressure distribution based on pressures detected by the plurality of pressure sensors, and spatial positions detected by the one or more position sensors.

**4.** The robot system of any one of claims 1 to 3, wherein the tactile information is one of a degree of smoothness and a degree of friction.

**5.** The robot system of any one of claims 1 to 4, wherein the sensible tactile information is sensed information defined so that a degree of sense by the operator changes corresponding to a degree of tactile in the tactile information.

**6.** The robot system of claim 5, wherein the sensed information is one of visual information, hearing information, and vibration, and the sensible tactile information presenting part is one of a display, a speaker, and an actuator.

**7.** The robot system of any one of claims 1 to 6, wherein,
the manipulator is a master arm, and an arm including the hand of the robot is a slave arm, and
the slave arm is manipulated by the master arm.

FIG. 1

FIG. 2

(a)

(b)

FIG. 3

(a)

(b)

(c)

FIG. 4

(a)

(b)

(c)

(a)

sx=0. 75

FREQUENCY

0

1  2  3

PRESSURE LEVEL

(b)

sx=0. 87

FREQUENCY

0

1  2  3

PRESSURE LEVEL

(c)

sx=0

FREQUENCY

0

1  2  3

PRESSURE LEVEL

FIG. 5

15

| STANDARD DEVIATION | SENSING LEVEL |
|---|---|
| $0.8 \leq sx \leq 1$ | 5 |
| $0.6 \leq sx < 0.8$ | 4 |
| $0.4 \leq sx < 0.6$ | 3 |
| $0.2 \leq sx < 0.4$ | 2 |
| $0 \leq sx < 0.2$ | 1 |

# FIG. 6

FIG. 7

(a)

(b)

FIG. 8

## INTERNATIONAL SEARCH REPORT

| | International application No. |
|---|---|
| | PCT/JP2016/003067 |

**A. CLASSIFICATION OF SUBJECT MATTER**
*B25J3/00*(2006.01)i, *B25J13/00*(2006.01)i, *B25J19/06*(2006.01)i

According to International Patent Classification (IPC) or to both national classification and IPC

**B. FIELDS SEARCHED**

Minimum documentation searched (classification system followed by classification symbols)
B25J3/00, B25J13/00, B25J19/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 6-110543 A (Nippon Telegraph and Telephone Corp.), 22 April 1994 (22.04.1994), paragraphs [0008] to [0018]; fig. 1 to 9 (Family: none) | 1-7 |
| A | JP 62-87154 A (Amada Co., Ltd.), 21 April 1987 (21.04.1987), entire text; all drawings (Family: none) | 1-7 |

☐ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 September 2016 (13.09.16) | 27 September 2016 (27.09.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**EP 3 342 545 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009282720 A
- JP 2014145717 A